Europäisches Patentamt

European Patent Office

Office européen des brevets

(19)

(11) **EP 0 728 304 B1**

(12) **EUROPÄISCHE PATENTSCHRIFT**

(45) Veröffentlichungstag und Bekanntmachung des Hinweises auf die Patenterteilung:
**10.06.1998 Patentblatt 1998/24**

(21) Anmeldenummer: **94931511.3**

(22) Anmeldetag: **07.11.1994**

(51) Int Cl.$^6$: **G01N 25/00**, G01K 7/36

(86) Internationale Anmeldenummer:
**PCT/DE94/01311**

(87) Internationale Veröffentlichungsnummer:
**WO 95/13532 (18.05.1995 Gazette 1995/21)**

(54) **VERFAHREN ZUR MESSUNG DER TEMPERATUR VON METALLISCHEN WERKSTÜCKEN UND IHRES FESTSTOFFANTEILS IM TEILERSTARRTEN ZUSTAND**

PROCESS FOR MEASURING THE TEMPERATURE OF METALLIC WORKPIECES AND THEIR SOLIDS CONTENT IN A PARTIALLY SOLIDIFIED STATE

PROCEDE DE MESURE DE LA TEMPERATURE DE PIECES METALLIQUES ET DE LEUR TENEUR EN COMPOSES SOLIDES A L'ETAT PARTIELLEMENT SOLIDIFIE

(84) Benannte Vertragsstaaten:
**AT BE CH DE ES FR GB IT LI NL SE**

(30) Priorität: **09.11.1993 DE 4338200**

(43) Veröffentlichungstag der Anmeldung:
**28.08.1996 Patentblatt 1996/35**

(73) Patentinhaber: **EFU GESELLSCHAFT FÜR UR-/UMFORMTECHNIK mbH**
**D-52152 Simmerath (DE)**

(72) Erfinder: **CREMER, Ralf**
**D-52074 Aachen (DE)**

(74) Vertreter: **König, Werner, Dipl.-Ing.**
**Habsburgerallee 23-25**
**52064 Aachen (DE)**

(56) Entgegenhaltungen:
**WO-A-82/02953**   **DD-A- 291 422**

- **GIESSEREI 80, NR. 4, Technische Umschau, veröffentlicht 22 Februar 1993, Seiten 111 - 112 U.PÄTZOLD ET AL. 'Bestimmung des Feststoffanteiles in Aluminiumlegierungen beim Thixogiessen durch neuen Wirbelstromsensor'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft ein Verfahren zur Messung der Temperatur von metallischen Werkstücken und ihres Feststoffanteils im teilerstarrten Zustand sowie eine zur Durchführung des Verfahrens nach einem der vorgehenden Ansprüche geeignete Vorrichtung mit einer Treiberspule, einer Meßspule und einer elektronischen Einheit zum Vergleichen der Signale von Treiberspule und Meßspule.

Bei verschiedenen Fertigungsverfahren sind möglichst genaue Kenntnisse über die Temperatur oder den inneren Gefügezustand eines metallischen Werkstükkes erforderlich. Während bei Verfahren zur Verarbeitung oder Veredelung metallischer Werkstoffe im festen Zustand (z.B.: Wärmebehandlung, Umformung, induktives Härten oder dgl.) vor allem die Temperatur und die damit verbundenen Phasenumwandlungen von Bedeutung sind, kommt es bei der Formgebung im teilerstarrten Zustand (z.B.: Thixogießen, Thixoschmieden) neben der Temperaturbestimmung vor allem auf die genaue Einhaltung der geforderten Anteile fester und flüssiger Phasen an.

Gegenstand der Erfindung ist ein Verfahren zur Messung eines in Abhängigkeit von der elektrischen Leitfähigkeit des metallischen Werkstückes stehenden Signals und dessen geeignete Auswertung, so daß Aussagen über die Temperatur oder über die Anteile fester und flüssiger Phasen beim teilweisen Aufschmelzen gewonnen werden können.

Unterhalb der Solidustemperatur besteht bei Metallen ein direkter Zusammenhang zwischen der elektrischen Leitfähigkeit und der Temperatur. Zwischen der Solidus- und der Liquidustemperatur können die Anteile fester und flüssiger Phasen in der Regel nicht über eine Temperaturmessung erfaßt werden, da beim Aufschmelzen bzw. Erstarren die Temperatur solange konstant bleiben kann, bis die entsprechende Schmelzwärme zugeführt oder abgeführt wurde. Hinzu kommt, daß die Temperatur des Phasenüberganges von der Aufheiz- bzw. Abkühlrate beeinfluß wird. Die elektrische Leitfähigkeit ist jedoch unter anderem gerade auch von den Anteilen fester und flüssiger Phasen abhängig und kann somit zur Ermittlung derselben genutzt werden.

Es ist bekannt (Gießerei 80(1993) Nr. 4/22. Februar, S. 111, 112), daß eine berührungslose Messung der elektrischen Leitfähigkeit auch innerhalb des Solidus-Liquidus-Intervalls Aufschluß über die Phasenübergänge und die Anteile fester und flüssiger Phase im zu untersuchenden Werkstück geben kann.

Für die Erwärmung wird dort offenbar eine gesonderte Treiberspule verwendet. Bei induktiver Erwärmung des Werkstückes muß die Erwärmungseinrichtung zur Messung abgeschaltet werden, da das durch die Erwärmungsleistung induzierte Feld die Messung verfälschen würde. Bei der genannten Meßfrequenz von 1 kHz beschränkt sich die Messung wegen des Skineffektes auf einen oberflächennahen Bereich des Werkstückes.

Die Aufgabe der vorliegenden Erfindung besteht nun darin, ein Verfahren anzugeben und eine zu seiner Durchführung geeignete Vorrichtung zu schaffen, um vor allem die vorstehenden Nachteile zu vermeiden und eine einfache und zuverlässige Lösung zu finden.

Diese Aufgabe wird erfindungsgemäß bei einem Verfahren der eingangs genannten Art dadurch gelöst,

daß den Werkstücken ein Treibersignal = Referenzsignal aufgegeben wird, welches in einer nahe dem Werkstück befindlichen Meßspule zu einem Meßsignal führt, das mit dem Treibersignal verglichen wird,
das Treibersignal von einer zur Erwärmung der Werkstücke dienenden und diese umschließenden Induktionsspule induziert wird,
eine Phasenverschiebung zwischen diesen beiden Signalen und/oder ein Quotient aus den jeweils amplitudenbezüglichen Werten der beiden Signale bestimmt wird,
eine Eichkurve betreffend die Abhängigkeit zwischen der Phasenverschiebung und/oder dem Wert des Quotienten einerseits und der Temperatur sowie dem Feststoffanteil bei einem solchen Werkstück andererseits nach herkömmlichen Methoden erstellt wird und
schließlich bei den weiteren solchen Werkstücken aus der Phasenverschiebung und/oder dem Wert des Quotienten über die Eichkurve die Temperatur und der Feststoffanteil ermittelt werden.

Das erfindungsgemäße Verfahren erfaßt den Zustand des gesamten induktiv erwärmten Gutes auch während einer induktiven Erwärmung. In diesem Fall bilden die Erwärmungsspule = Treiberspule und eine Meßspule zusammen mit dem Werkstück ein gekoppeltes Spulensystem. Die elektromagnetischen Feldkomponenten des von der Erwärmungsspule erzeugten elektromagnetischen Feldes erfahren beim Durchgang durch das Werkstück eine Dämpfung und eine Phasenverschiebung, welche von der elektrischen Leitfähigkeit und der Permeabilität (nur bei ferromagnetischem Material) des Werkstückes bestimmt wird. Die Phasenverschiebung und/oder Dämpfung kann durch geeignete mathematisch-schaltungstschnische Verknüpfung der beiden Signale ermittelt werden, die an der Treiberspule und an der Meßspule abgegriffen werden können. Die Amplitude des an der Meßspule abgegriffenen Signals wird vor allem durch die Erwärmungsleistung und die Dämpfung bestimmt.

Es wurde festgestellt, daß die Phasenverschiebung und der Wert des Quotienten der beiden Signale nur von den elektrischen und magnetischen Werkstoffdaten des Werkstückes aber - im Gegensatz zur Amplitude - nicht von der induzierten induktiven Erwärmungsleistung bestimmt wird. Demzufolge erfolgt beim erfindungsgemäßen Verfahren die mathematischschaltungstechnische Verknüpfung des Treibersignals und des Meßspulensignals so, daß das gewonnene Meßsignal nur von der Phasenverschiebung zwischen dem Treibersignal und dem Maßspulensignal und/oder dem Wert des Quotienten aus Treibersignal und Meßspulensignal abhängt.

Nach Aufnahme einer legierungs- und geometriespezifischen Eichkurve können nach dem erfindungsgemäßen Verfahren die Temperatur des Werkstückes bis zur Solidustemperatur oder die Anteile fester und flüssiger Phasen zwischen der Solidus- und Liquidustemperatur bestimmt werden. Da das Meßsignal unabhängig von der Erwärmungsleistung ist, kann es bei Bedarf direkt zum Aufbau einer kontinuierlich arbeitenden Regelung des Erwärmungsvorganges genutzt werden.

Eine Weiterbildung des erfindungsgemäßen Verfahrens kann darin bestehen, daß der Quotient aus den Effektivwerten der beiden Signale bestimmt wird.

Eine Weiterbildung des erfindungsgemäßen Verfahrens kann darin bestehen, daß die Einspeisefrequenz des Treibersignals zwischen 50 Hz und 10 kHz liegt. Die Integration des Meßverfahrens in bestehende Erwärmungsanlagen ist somit ohne weiteres möglich.

Eine Weiterbildung des erfindungsgemäßen Verfahrens kann darin bestehen, daß die Treiberfrequenz konstant gehalten wird. Hierdurch kann eine werkstückspezifische Eichkurve in anderen induktiven Erwärmungsanlagen mit fester Einspeisefrequenz übernommen werden.

Eine Weiterbildung des erfindungsgemäßen Verfahrens kann darin bestehen, daß als Meßspulensignal die in der Meßspule induzierte Spannung gewählt wird. Durch diese Maßnahme und eine hochohmige Weiterverarbeitung dieser Spannung wird die Leistung im Sensorkreis nahezu konstant und verlustlos gehalten. Die Rückwirkungen des Sensorkreises auf das Treibersignal sowie Temperatureinflüsse im Meßspulenkreis sind daher vernachlässigbar.

Eine Weiterbildung des erfindungsgemäßen Verfahrens kann darin bestehen, daß das Referenzsignal aus dem Strom der Erwärmungsspule abgeleitet wird. Hierdurch wird der Einfluß der Impedanz, welche aus Treiberspule, Werkstück und Meßspule gebildet wird, auf das Meßsignal eliminiert.

Die Aufgabe wird erfindungsgemäß durch eine Vorrichtung der eingangs genannten Art gelöst, die sich dadurch auszeichnet, daß die Treiberspule als die Werkstücke umschließende und erwärmende Induktionsspule ausgebildet ist und daß die Meßspule gegen mechanische Beanspruchung und gegen mechanisches Verrücken in einem feuerfesten Material eingebettet und auf einem mit dem zu messenden Werkstück im Spulenfeld der Induktionsspule positionierbaren Träger montiert ist. Es ist also keine zusätzliche Kühlung der Meßspule notwendig.

Eine vorteilhafte Weiterbildung der erfindungsgemäßen Vorrichtung kann darin bestehen, daß die Meßspule in einer Keramikplatte oder in einem Keramikring eingebettet auf der Stirnfläche eines zur Formgebung benutzten Werkzeugs angebracht ist. Insbesondere ist eine solche Vorrichtung für hohe mechanische Beanspruchung der Meßspule geeignet.

Eine vorteilhafte Weiterbildung der erfindungsgemäßen Vorrichtung kann darin bestehen, daß die Meßspule in einem Träger integriert ist. Hierdurch ist ein genaues und reproduzierbares Positionieren von Werkstück und Meßspule im Treiberfeld zueinander möglich.

Eine vorteilhafte Weiterbildung der erfindungsgemäßen Vorrichtung kann darin bestehen, daß die Meßspule in einem elektrischen Isolierkörper möglichst nahe an der Stirnfläche des Werkstückes angeordnet ist. Hierdurch wird eine hohe Auflösung des Meßsignals erreicht.

Eine vorteilhafte Weiterbildung der erfindungsgemäßen Vorrichtung kann darin bestehen, daß die Meßspule gegenüber dem Werkstück thermisch isoliert ist. Dabei ist keine zusätzliche Kühlung für die Meßspule erforderlich.

Eine vorteilhafte Weiterbildung der erfindungsgemäßen Vorrichtung kann darin bestehen, daß die Windungen der Meßspule innerhalb des Profils des Werkstückes angeordnet sind. Dies beeinflußt die Auflösung des Meßsignals vorteilhaft.

Eine vorteilhafte Weiterbildung der erfindungsgemäßen Vorrichtung kann darin bestehen, daß die Windungen in konstantem Abstand von der Kontur der Stirnfläche des Werkstückes innerhalb des Profils dieser Stirnfläche angeordnet sind. Auf diese Weise wird es möglich, bestimmte Werkstückbereiche zu analysieren und dabei die Auflösung des Meßsignals günstig zu beeinflussen.

Eine vorteilhafte Weiterbildung der erfindungsgemäßen Vorrichtung kann darin bestehen, daß die Meßspule durch eine einfache oder durch eine mehrfache ringförmige Drahtschleife gebildet ist. Bei dieser einfachen und kompakten Bauweise ist eine Integration der Maßspule in bestehende induktive Erwärmungseinrichtungen besonders leicht möglich.

Im folgenden Teil der Beschreibung werden einige Ausführungsformen der Erfindung anhand von Zeichnungen beschrieben. Es zeigt:

Fig. 1: eine prinzipielle Anordnung der signalerfassenden Elemente in einer induktiven Erwärmungsanlage zur Durchführung des erfindungsgemäßen Verfahrens;

Fig. 2: ein Blockschaltbild des Meßverfahrens, welches in einer induktiven Erwärmungsanlage integriert ist;

Fig. 3: einen prinzipiellen Verlauf des Referenzsignals und des Meßspulensignals bei einem konstanten Zustand des Werkstückes über die Zeit aufgetragen;

Fig. 4: einen Meßschrieb, in welchem das Meßsignal und im Vergleich dazu die Temperatur in einem Werkstück während dessen Erwärmung über die Zeit aufgetragen ist;

Fig. 5: eine erste Ausführungsform der erfindungsgemäßen Meßspule im Achsialschnitt sowie in Draufsicht;

Fig. 6: eine weitere Ausführungsform der erfindungsgemäßen Meßspule im Achsialschnitt sowie in Draufsicht;

Fig. 7: eine Ausführungsform der Meßspule, bei der diese in einem Träger aus Isoliermaterial eingebettet ist;

Fig. 8: eine für hohe mechanische Beanspruchung geeignete Ausführungsform der erfindungsgemäßen Meßspule, bei der diese in einer Keramikplatte eingebettet und auf einem Träger befestigt ist;

Fig. 9: eine weitere für hohe mechanische Beanspruchung geeignete Ausführungsform, bei der die Maßspule in einem Ring aus Keramik eingebettet und auf einem Träger befestigt ist.

Beispiel 1:

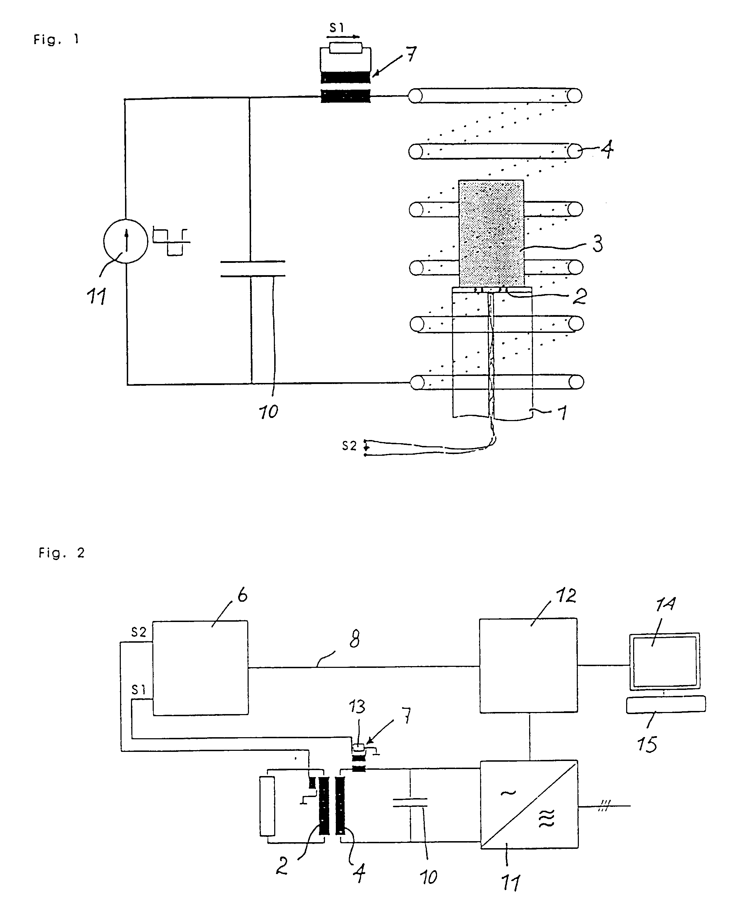

Fig. 1 zeigt prinzipiell die Anordnung der signalerfassenden Elemente in einer induktiven Erwärmungsanlage zur Durchführung des erfindungsgemäßen Verfahrens. Ein Träger 1 dient zur Aufnahme einer Meßspule 2 und zur genauen Positionierung der Meßspule 2 an der Stirnfläche eines Werkstückes 3 und/oder zur Positionierung des Werkstückes 3 mit Meßspule 2 im Spulenfeld einer Treiberspule 4. Bis zur Liquidustemperatur des Werkstückes 3 ändert das Trägermaterial seine stoffspezifischen elektrischen und magnetischen Eigenschaften nur schwach im Vergleich zu den elektromagnetischen Eigenschaften des Werkstückes 3. Welches Trägermaterial eingesetzt wird, hängt entscheidend von der mechanischen Beanspruchung des Trägers 1 mit Meßspule 2 ab.

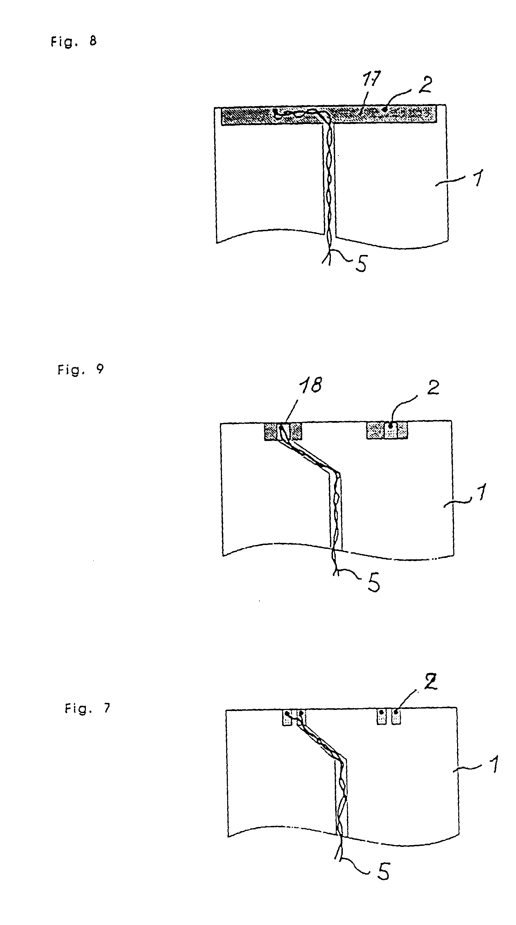

Im vorliegenden Fall ist entsprechend Fig. 7 die Meßspule 2 in elektrisch isolierendes temperaturfestes Material in einer Nut im Träger 1 eingebettet, welcher ebenfalls aus isolierendem Material, z.B. gepreßtem Aluminiumoxid, besteht. Die Meßspule 2 ist mit einer Zuleitung 5 versehen.

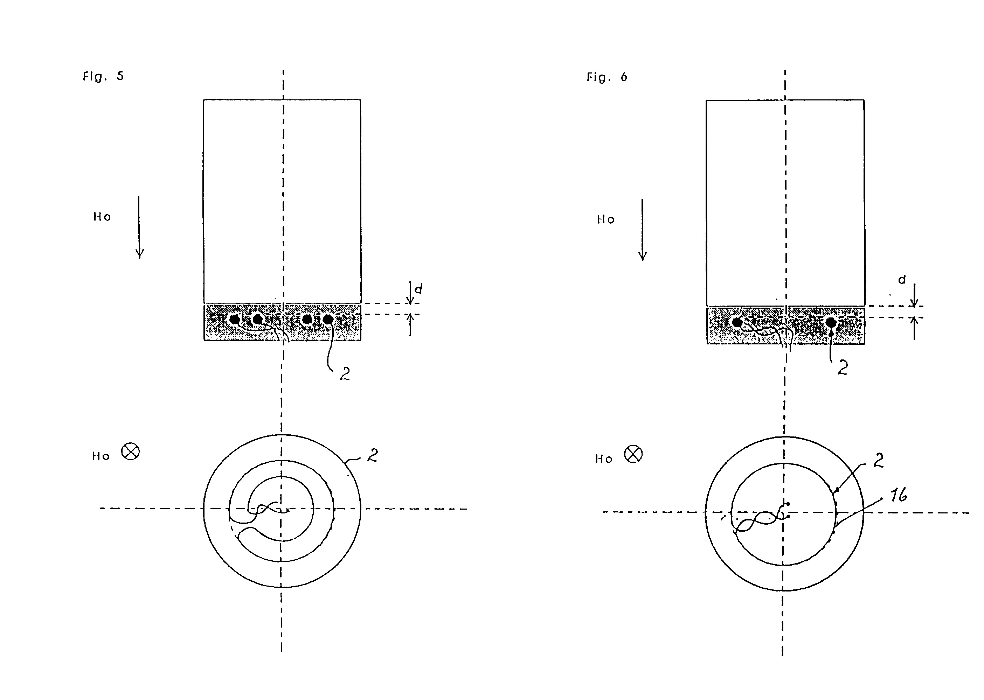

Die Meßspule 2 gemäß Fig. 5 besteht aus zwei Windungen, die so in einer Ebene im Isoliermaterial verlegt sind, daß die induzierten Spannungen einer jeden Windung sich teilweise kompensieren. Hierdurch wird das Meßspulensignal für ein hier gewähltes Phasenvergleichsmeßgerät 6 (Fig. 2) günstig beeinflußt.

Die Zuleitungen 5 der Meßspule 2 sind durch Glasseidenschlauch isoliert und verdrillt durch eine zentrische Bohrung durch den Träger 1 an die Meßspule 2 geführt. Die Anordnung aus Träger 1, Meßspule 2 und Zuleitung 5 ist in horizontaler Richtung beweglich auf einer geeigneten Hebevorrichtung montiert. Das Werkstück 3, z.B. ein AlSi7Mg-Bolzen, wird auf dem Träger 1 mit Meßspule 2 positioniert und an eine definierte Stelle in dem elektromagnetischen Feld der zur Erwärmung des Werkstückes 3 dienenden Treiberspule 4 gebracht. Dabei soll der Abstand d (Fig. 5 und Fig. 6) zwischen Werkstück 3 und Meßspule 2 so klein sein, wie es die Isolationserfordernisse erlauben, um eine möglichst hohe Auflösung des Meßsignals zu erzielen. Auch wird die Auflösung des Meßsignals erheblich verbessert dadurch, daß die Kontur der Meßspule 2 nicht über die Stirnfläche des Werkstückes 3 hinausragt (Fig. 5 und Fig. 6). Zudem soll die Außenkontur der Meßspule 2 in gleichem Abstand von der Stirnflächenkontur des Werkstückes 3 verlaufen.

Nach dem Induktionsgesetz wird bei eingeschalteter Treiberspule 4 eine Spannung S2 in der Meßspule 2 induziert. Das Referenzsignal S1 des Treiberspulenstroms wird mit einer Strommeßzange 7 (Fig. 1) erfaßt und in ein Spannungssignal umgeformt. Das Referenzsignal S1 und das in der Meßspule 2 induzierte Spannungssignal S2 werden jeweils über eine Koaxialleitung an das Phasenvergleichsmeßgerät 6 = Lock-in-Verstärker (Fig. 2) geführt. Mit Hilfe des Lock-in-Verstärkers 6 wird das Meßsignal Um 8 gebildet und als Gleichspannung ausgegeben.

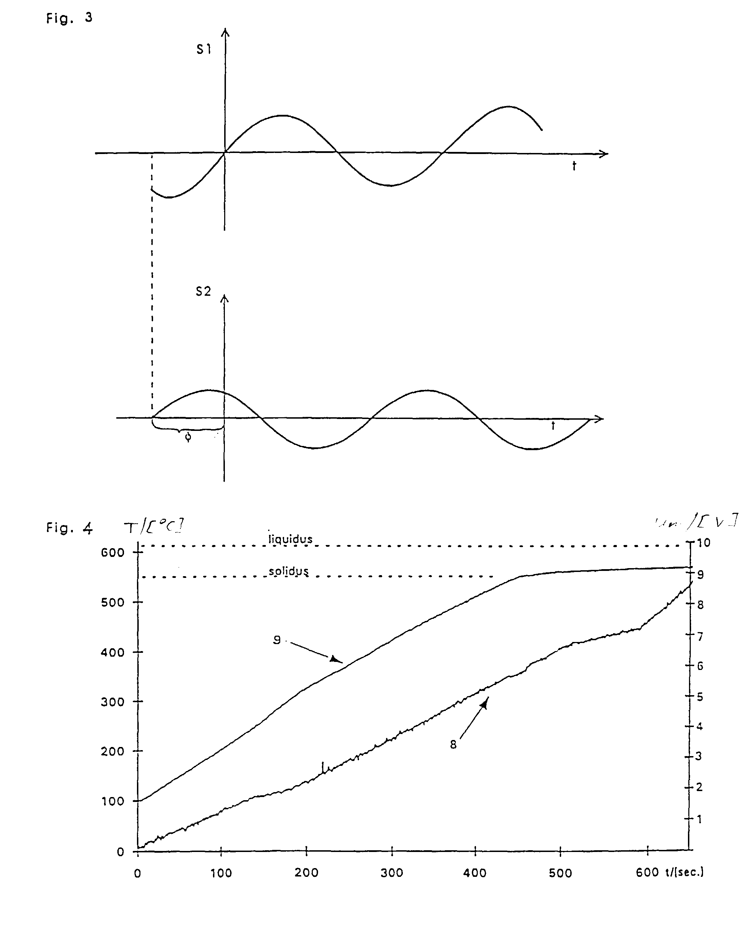

In Fig. 4 ist ein Meßschrieb dargestellt. Die Kurve 8 zeigt über der Erwärmungszeit eines Werkstückes 3 das Meßsignal Um in Volt, welches nach dem erfindungsgemäßen Verfahren gewonnen wurde. Im Vergleich dazu zeigt die Kurve 9 die Temperatur des Werkstückes 3 in °C, welche mit einem Ni-CrNi-Thermoelement gemessen wurde. Die Kurve 9 ist demnach eine Eichkurve für die Werkstückgegebenheiten, die es ermöglicht, dem Meßsignal Um gemäß Kurve 8 bei gleichen Werkstückgegebenheiten eine Temperatur und darüber hinaus im Bereich zwischen Solidus- und Liquiduslinie eine Angabe über den Feststoffanteil des Werkstückes 3 zu entnehmen.

Beispiel 2:

In Fig. 2 ist ein Blockschaltbild mit elektrischem Ersatzschaltbild der Meßanordnung aus Beispiel 1 zusammen mit den signalbildenden Einheiten dargestellt. Es liegt ein Parallelschwingkreis vor, der eine Treiberspule 4 und einen Schwingkreiskondensator 10 hat und von einem Umrichter 11 gespeist wird, welcher seinerseits von einem Prozeßleitsystem 12 angesteuert wird. Das Werkstück 3 mit Treiberspule 4 wird als Transformator betrachtet, welcher primärseitig vom Treiberspulenstrom gespeist wird und sekundärseitig durch eine ohmsche Last belastet ist. Am Werkstück 3 wird das Meßspulensignal S2 induktiv erfaßt. Das Referenzsignal S1 wird mit Hilfe einer Strommeßzange 7 abgegriffen und über einen ohmschen Meßwiderstand 13 zu einem Spannungssignal umgeformt. Diese beiden Signale werden an den Eingang des Phasenvergleichsmeßgerätes 6 geführt. Die Ausgabe des Meßsignals erfolgt linear in Abhängigkeit von der Phasenverschiebung der Eingangssignale in einem Spannunsbereich von 0 bis 10 Volt. Das Meßsignal Um wird an das Prozeßleitsystem 12 geführt. Mit Hilfe von abgespeicherten Eichkurven wird der Erwärmungsprozeß auf einem Monitor 14 des Prozeßleitsystems 12 beobachtet und/oder über eine Tastatur 15 des Prozeßleitsystems 12 beeinflußt oder auch über das Prozeßleitsystem 12 geregelt.

Alternativ zur Messung der Phasenverschiebung kann auch die Dämpfung gemessen werden. Dazu werden die beiden Signale S1 und S2 in einem geeigneten Meßgerät in ihre jeweiligen Effektivwerte umgeformt und anschließend untereinander dividiert. In diesem Fall erfolgt die Ausgabe des Meßsignals 8 in Abhängigkeit von dem Quotientenwert in einem Spannungsbereich von 0 bis 10 Volt.

Beispiel 3:

Wie in Beispiel 1 und 2 beschrieben, kann das Meßverfahren mit verschiedenen Meßspulenkonstruktionen durchgeführt werden. In Fig. 6 ist eine Meßspule 2 dargestellt, die sich durch eine besonders einfache Konstruktion auszeichnet. Sie besteht lediglich aus einer Windung 16, welche konzentrisch zur Stirnfläche des Werkstückes 3 angeordnet ist.

Beispiel 4:

In Fig. 8 und in Fig. 9 sind Ausführungsformen der Meßspule 2 dargestellt, welche für die Realisierung des Meßverfahrens unter besonders hoher mechanischer Belastung von Träger 1 und Meßspule 2 geeignet ist. Der Träger 1 mit Meßspule 2 ist auf der Kolbenstirnfläche einer Druckgießmaschine (Thixopresse) angebracht. In diesem Fall besteht das Trägermaterial aus nichtmagnetischem Stahl (4828, 4878 o.ä.) oder aus Keramik. Das erwärmte Werkstück 3 wird zusammen mit dem Träger 1 und der entsprechend druck- und temperaturfest eingebetteten Meßspule 2 in die Gießkammer bewegt. Die Meßspule 2 ist in eine Keramikplatte 17 eingebacken oder eingebettet. Diese ist auf dem Träger 1 befestigt (geklebt, eingeschrumpft o.ä.). Eine andere Möglichkeit besteht darin, die Meßspule 2 in einen Keramikring 18 mit Hohlprofil (Fig. 9) einzubetten. Dieser Keramikring 18 ist in eine passende Nut in der Stirnfläche des Trägers 1 eingelegt und befestigt.

Bezugszeichenliste:

1   Träger
2   Meßspule = Sensorspule
3   Werkstück
4   Treiberspule
5   Zuleitung
6   Phasenvergleichsmeßgerät
7   Strommeßzange
8   Kurve (Meßsignal)
9   Kurve (Temperatur des Werkstückes)
10  Schwingkreiskondensator
11  Umrichter
12  Prozeßleitsystem
13  Meßwiderstand
14  Monitor
15  Tastatur
16  Windung
17  Keramikplatte
18  Keramikring

**Patentansprüche**

1.  Verfahren zur Messung der Temperatur von metallischen Werkstücken und ihres Feststoffanteils im teilerstarrten Zustand,
    dadurch gekennzeichnet, daß

    den Werkstücken ein Treibersignal, d.h. Referenzsignal, aufgegeben wird, welches in einer nahe dem Werkstück befindlichen Meßspule zu einem Meßsignal führt, das mit dem Treibersignal verglichen wird,

    das Treibersignal von einer zur Erwärmung der Werkstücke dienenden und diese umschließenden Induktionsspule induziert wird,

    eine Phasenverschiebung zwischen diesen beiden Signalen und/oder ein Quotient aus den jeweils amplitudenbezüglichen Werten der beiden Signale bestimmt wird,

    eine Eichkurve betreffend die Abhängigkeit zwischen der Phasenverschiebung und/oder dem Wert des Quotienten einerseits und der Temperatur sowie dem Feststoffanteil bei einem solchen Werkstück andererseits nach herkömmlichen Methoden erstellt wird und

    schließlich bei den weiteren solchen Werkstücken aus der Phasenverschiebung und/oder dem Wert des Quotienten über die Eichkurve die Temperatur und der Feststoffanteil ermittelt werden.

2.  Verfahren nach Anspruch 1, dadurch gekennzeichnet, daß der Quotient aus den Effektivwerten der beiden Signale bestimmt wird.

3.  Verfahren nach Anspruch 1 oder 2, dadurch gekennzeichnet, daß es sich bei den Werkstücken um metallische Blöcke aus einer thixotropen Legierung handelt, die im teilerstarrten Zustand weiterverarbeitet werden.

4. Verfahren nach einem der vorhergehenden Ansprüche, dadurch gekennzeichnet, daß die Einspeisefrequenz des Treibersignals zwischen 50 Hz und 10 kHz liegt.

5. Verfahren nach einem der vorhergehenden Ansprüche, dadurch gekennzeichnet, daß die Treiberfrequenz konstant gehalten wird.

6. Verfahren nach einem der vorhergehenden Ansprüche, dadurch gekennzeichnet, daß als Meßspulensignal die in der Meßspule induzierte Spannung gewählt wird.

7. Verfahren nach einem der vorhergehenden Ansprüche, dadurch gekennzeichnet, daß das Referenzsignal aus dem Strom der Erwärmungsspule abgeleitet wird.

8. Zur Durchführung des Verfahrens nach einem der vorhergehenden Ansprüche geeignete Vorrichtung mit einer Treiberspule (4) und einer Meßspule (2), dadurch gekennzeichnet,
daß eine elektronische Einheit zum Vergleichen der Signale von Treiberspule (4) und Meßspule (2) vorgesehen ist,
daß die Treiberspule (4) als die Werkstücke (3) umschließende und erwärmende Induktionsspule ausgebildet ist und daß die Meßspule (2) gegen mechanische Beanspruchung und gegen mechanisches Verrücken in einem feuerfesten Material eingebettet und auf einem mit dem zu messenden Werkstück (3) im Spulenfeld der Induktionsspule positionierbaren Träger (1) montiert ist.

9. Vorrichtung nach Anspruch 7, dadurch gekennzeichnet, daß die Meßspule (2) in einer Keramikplatte (17) oder in einem Keramikring (18) eingebettet auf der Stirnfläche eines zur Formgebung benutzten Werkzeugs angebracht ist.

10. Vorrichtung nach Anspruch 8 oder 9, dadurch gekennzeichnet, daß die Meßspule (2) in einem Träger (1) integriert ist.

11. Vorrichtung nach einem der Ansprüche 8 bis 10, dadurch gekennzeichnet, daß die Meßspule (2) in einem elektrischen Isolierkörper möglichst nahe an der Stirnfläche des Werkstückes (3) angeordnet ist.

12. Vorrichtung nach einem der Ansprüche 8 bis 11, dadurch gekennzeichnet, daß die Meßspule (2) gegenüber dem Werkstück (3) thermisch isoliert ist.

13. Vorrichtung nach einem der Ansprüch 8 bis 12, dadurch gekennzeichnet, daß die Windungen der Meßspule (2) innerhalb des Profils des Werkstükkes (3) angeordnet sind.

14. Vorrichtung nach Anspruch 12, dadurch gekennzeichnet, daß die Windungen in konstantem Abstand von der Kontur der Stirnfläche des Werkstükkes (3) innerhalb des Profils dieser Stirnfläche angeordnet sind.

15. Vorrichtung nach Anspruch 12, dadurch gekennzeichnet, daß die Meßspule (2) durch eine einfache oder durch eine mehrfache ringförmige Drahtschleife gebildet ist.

**Claims**

1. A process for measuring the temperature of metallic workpieces and their solid content in a partially solidified state
characterised in that

the workpieces are sent a driving signal, i.e. a reference signal which turns into a measurement signal in an exploring coil situated near the workpiece; the measurement signal is compared with the driving signal,

the driving signal is induced by an induction coil which serves to heat and surround the workpieces,

a phase shift between these two signals is defined and/or a quotient is defined from the amplitude-related values of the two signals,

a calibration curve relating to the inter-dependence between the phase shift and/or the value of the quotient on the one hand and the temperature and the percentage of solid matter in this type of workpiece on the other hand is plotted applying conventional methods, and

finally, in other workpieces of this type, the temperature and the percentage of solid matter are calculated from the phase shift and/or the value of the quotient by referring to the calibration curve.

2. A process in accordance with claim 1, characterised in that the quotient is determined from the effective values of the two signals.

3. A process in accordance with claims 1 or 2, characterised in that the workpieces are metallic blocks made from a thixotropic alloy, which are processed in a partially solidified state.

4. A process in accordance with one of the foregoing claims, characterised in that the incoming frequency of the driving signal lies between 50 Hz and 10 Hz.

5. A process in accordance with one of the foregoing claims, characterised in that the driving frequency is kept constant.

6. A process in accordance with one of the foregoing claims, characterised in that the voltage induced in the exploring coil is selected to serve as the exploring coil signal.

7. A process in accordance with one of the foregoing claims, characterised in that the reference signal is derived from the heating coil current.

8. A device which is suitable for performing the process in accordance with one of the foregoing claims, comprising a driving coil (4) and an exploring coil (2), characterised in

   that an electronic unit for comparing the signals from the driving coil (4) and the exploring coil (2) is provided;
   that the driving coil (4) serves as the induction coil which surrounds and heats the workpieces (3),
   and that the exploring coil (2) is embedded in fireproof material to safeguard it against mechanical stress and mechanical displacement and is mounted on a support (1) holding the workpiece to be measured [3], which can be positioned with the measurement workpiece (3) within the field of the induction coil.

9. A device in accordance with claim 7, characterised in that the exploring coil (2) is attached to a ceramic plate (17) or to a ceramic ring (18), embedded in the front of a tool used for shaping.

10. A device in accordance with claims 8 or 9, characterised in that the exploring coil (2) is integrated within a support (1).

11. A device in accordance with one of claims 8 to 10, characterised in that the exploring coil (2) is arranged within an electrical insulator, positioned as closely as possible to the front of the workpiece (3).

12. A device in accordance with one of claims 8 to 11, characterised in that the exploring coil (2) is thermally insulated against the workpiece (3).

13. A device in accordance with one of claims 8 to 12, characterised in that the turns of the exploring coil (2) are arranged within the profile of the workpiece (3).

14. A device in accordance with claim 12, characterised in that the turns are arranged at a constant distance from the contour of the front of the workpiece (3) within the profile of this front.

15. A device in accordance with claim 12, characterised in that the exploring coil (2) is formed by a single or a multiple ring-shaped wire coil.

## Revendications

1. Procédé de mesure de la température de pièces d'oeuvres métallique et de leur pourcentage en matériau solide dans un état partiellement solidifié, caractérisé en ce

   qu'un signal d'attaque, c'est à dire un signal de référence, est transmis aux pièces d'oeuvre, ce signal provoquant un signal de mesure dans une bobine de mesure qui se trouve près de la pièce d'oeuvre; ce signal est comparé au signal d'attaque;

   que le signal d'attaque est induit par une bobine d'induction servant à l'échauffement de la pièce d'oeuvre et la recouvrant;

   qu'un déplacement de phases entre ces deux signaux et/ou un quotient issu des valeurs d'amplitude respectives de ces deux signaux est déterminé;

   qu'une courbe d'étalonnage concernant la dépendance entre le déplacement de phase et/ou la valeur du quotient d'une part, ainsi que la température et le pourcentage en matériaux solides dans une telle pièce d'oeuvre d'autre part, est établie d'après les méthodes courantes et,

   qu'enfin, la température et le pourcentage en matériaux solides des autres pièces d'oeuvres similaires sont calculés à partir du déplacement de phases et/ou de la valeur du quotient par le biais de la courbe d'étalonnage.

2. Procédé selon la revendication 1, caractérisé par le fait que le quotient est déterminé à partir des valeurs effectives des deux signaux.

3. Procédé selon la revendication 1 ou 2, caractérisé par le fait que les pièces d'oeuvres sont des blocs métalliques en alliage thixotrope qui sont transformées dans un état en partie solidifié.

4. Procédé selon l'une des revendications précédentes, caractérisé par le fait que la fréquence d'alimentation du signal d'attaque se situe entre 50 Hz et 10 kHz.

5. Procédé selon l'une des revendications précédentes, caractérisé par le fait que la fréquence d'attaque est maintenue à un niveau constant.

6. Procédé selon l'une des revendications précédentes, caractérisé par le fait que la tension induite dans la bobine de mesure est sélectionnée en tant que signal de bobine de mesure.

7. Procédé selon l'une des revendications précédentes, caractérisé par le fait que le signal de référence est déduit du courant de la bobine d'échauffement.

8. Dispositif propre pour la réalisation de la procédé selon l'une des revendications ci-dessus avec une bobine d'attaque (4), caractérisé en ce

   qu'une unité électronique pour la comparaison des signaux de la bobine d'attaque (4) et de la bobine de mesure (2) est prévue;

   que la bobine d'attaque (4) est conçue en tant que bobine d'induction recouvrant et échauffant les pièces d'oeuvre (3)

   et que la bobine de mesure (2) est insérée dans un matériau réfractaire pour une protection contre l'usure mécanique et un déplacement mécanique et est montée sur un support (1), qui peut être positionné, avec la pièce d'oeuvre à mesurer (3) sur le champ de bobine de la bobine d'induction.

9. Dispositif selon la revendication 7, caractérisé par le fait que la bobine de mesure (2) est insérée dans une plaque en céramique (17) ou dans un anneau en céramique (18) et est placé sur la surface frontale d'un outil utilisé pour le formage.

10. Dispositif selon la revendication 8 ou 9, caractérisé par le fait que la bobine de mesure (2) est intégrée sur un support (1).

11. Dispositif selon l'une des revendications 8 à 10, caractérisé par le fait que la bobine de mesure (2) est disposée dans un corps à isolation électrique aussi proche que possible de la surface frontale de la pièce d'oeuvre (3).

12. Dispositif selon l'une des revendications 8 à 11, caractérisé par le fait que la bobine de mesure (2) a une isolation thermique par rapport à la pièce d'oeuvre (3).

13. Dispositif selon l'une des revendications 8 à 12, caractérisée par le fait que les torsions de la bobine de mesure (2) sont disposées dans le profil de la pièce d'oeuvre (3).

14. Dispositif selon la revendication 12, caractérisé par le fait que les torsions sont disposées à raison d'une distance constante du contour de la surface frontale de la pièce d'oeuvre (3) dans le profil de cette surface frontale.

15. Dispositif selon la revendication 12, caractérisé par le fait que la bobine de mesure (2) est formée d'une boucle annulaire en fil d'acier simple ou multiple.

Fig. 1

Fig. 2

Fig. 3

Fig. 4

Fig. 6

Ho

Ho

Fig. 5

Ho

Ho

Fig. 8

Fig. 9

Fig. 7